# EUROPEAN PATENT SPECIFICATION

(11) **EP 1 521 798 B1**
(45) Date of publication and mention of the grant of the patent: **27.01.2010**
(21) Application number: 03718801.8
(22) Date of filing: 09.05.2003
(51) Int. Cl.: C08G 77/38, C08G 77/388

(54) **SUNSCREENS**
SONNENSCHUTZMITTEL
ECRANS SOLAIRES

(30) Priority: 16.07.2002 EP 02015849
(43) Date of publication of application: 13.04.2005
(73) Proprietor: DSM IP Assets B.V., 6411 TE Heerlen (NL)
(72) Inventor: BERG-SCHULTZ, Katja, CH-4303 Kaiseraugst (CH); HUBER, Ulrich, CH-8703 Erlenbach (CH)
(74) Representative: Schwander, Kuno
(86) International application number: PCT/EP2003/004892
(87) International publication number: WO 2004/007592

(56) References cited:
- EP-A- 0 709 080
- EP-A- 1 000 950
- EP-A- 1 081 140

## Description

The present invention relates to novel sunscreens on the basis of polysiloxanes, to their preparation and to their use, especially in formulations for the protection against harmful effects of sunlight.

There is a constantly increasing need for sunscreen protection agents in a population that is exposed to an increasing amount of damaging sunlight. Repetitive sun exposure can result in skin changes known as photoaged skin. The clinical changes that are seen in photoaged skin differ from those of normally aged skin in sunlight protected sites of the body. Among the damaging results of intensive sun exposure of the skin there is increased wrinkling, elastosis, pigmentary changes, precancerous and cancerous skin lesions.

Many sunscreening chemicals have been developed in the past protecting against the harmful effects of UVA (320 - 400 nm) and/or UVB (290 - 320 nm) wavelengths. These chemicals have been incorporated either alone or in combination with each other into cosmetic or pharmaceutical preparations which are widely known and used. However, these is a need for the development of even more potent sunscreening chemicals and preparations containing them as well as of easier and economically more attractive chemical syntheses thereof, especially in view of further depletion of the ozon layer of the earth to be expected with concurrent increasing doses of UVA and UVB and even shorter wavelengths (UVC) to be endured.

The present invention, therefore, provides new polysiloxane-based sunscreen compounds, a method for their preparation and cosmetic or pharmaceutical compositions containing them.

More particularly the new compounds of the present invention are substituted polysiloxanes containing a certain number of the following structural elements per molecule, viz.

one element of formula (H₃C)₃-Si- (I),

one element of formula -O-Si(CH₃)₃ (II),

2 to 200, preferably 5 to 80, elements in arbitrary order which are either identical or different from each other selected from the group consisting of formulae

-O-Si(CH₃)[CH(CH₃)R¹]- (IIIa),

-O-Si(CH₃)(CH₂-CH₂-R¹)- (IIIb),

-O-Si(CH₃)[C(=CH₂)R¹]- (IIIc),

and

-O-Si(CH₃)(CH=CH-R¹)- (IIId);

2 to 200, elements in arbitrary order which are either identical or different from each other selected from the group consisting of formulae

-O-Si(CH₃)[CH(CH₃)R²]- (IVa),

-O-Si(CH₃)(CH₂-CH₂-R²)- (IVb),

-O-Si(CH₃)[C(=CH₂)R²]- (IVc),

and

-O-Si(CH₃)(CH=CH-R²)- (IVd);

optionally 1 to 100, preferably 1 to 10, elements in arbitrary order which are either identical or different from each other selected from the group consisting of formulae

-O-Si(CH₃)[CH(CH₃)R³]- (Va),

-O-Si(CH₃)(CH₂-CH₂-R³)- (Vb),

-O-Si(CH₃)[C(=CH₂)R³]- (Vc),

and

-O-Si(CH₃)(CH=CH-R3)- (Vd);

and optionally 1 - 20 elements in arbitrary order of formula -O-SiH(CH₃)- (VI)
wherein R¹ is a UV light absorbing group;
R² is hydrogen or a lipophilic group;
R³ is a group which is able to form ionogenic or hydrogen bonds.

The term "in arbitrary order" means that there is no specific order or sequence of the elements of formulae III a - III d, IV a - IV d, V a - V d and VI in the polysiloxane molecule and that the order or sequence of the elements may vary from molecule to molecule.

Elements may be "identical" or "different from each other". This means that there may be present only one type of an element of formula IIIa - IIId and/or of formula IVa - IVd and/or of formula V a - V d wherein again each of the substituents R¹, R² and R³ may be identical or different from each other. Thus, all possible combinations of different elements as defined above are comprised by the present invention. For example the sunscreen molecules of the present invention may contain 1- 200 entities of a single type of UV.absorbing groups (chromophores), viz. only one type of UVA or UVB absorbing group. Alternatively, two or more different types of UVA and/or UVB absorbing groups may be present in the same molecule in arbitrary order. If protection against a broad wavelength spectrum is desired the use of different groups with different absorption maxima, including UVC absorbing groups, may be preferable.

Thus, e.g., a polysiloxane compound of formula

(H₃C)₃Si-[-O-Si(CH₃)(CH₂-CH₂-R¹)]₃₁-[-O-Si(CH₃)(C[=CH₂]-R²)]₁₃-O-Si(CH₃)₃

contains in arbitrary order 44 silyloxy groups, 31 of which being groups of formula IIIb with chromophores R¹ (R¹ being identical or different from each other), 13 of which being groups of formula IVc with hydrogen or lipophilic substitutents R² (R² being identical or different from each other).

The polysiloxane compounds of the present invention can be prepared by reacting a polymethylhydrosiloxane of the general formula 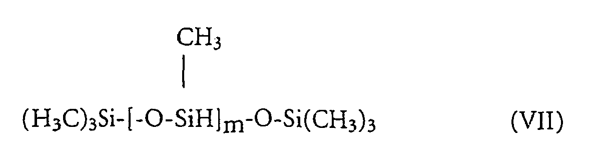 wherein m is an integer of 4 to 520, preferably of 10 to 200 and more preferably of 20 to 60,
with terminally unsaturated compounds of formulae

H₂C=CH-R¹ (VIIIa) and H₂C = CH - R² (VIIIb)

and/or HC ≡ C - R¹ (IXa) and/or HC =C - R² (IXb)
and optionally H₂C = CH - R³ (Xa) and/or HC ≡ C - R³ (Xb),
wherein R¹, R² and R³ are as defined above,
in the presence of a noble metal catalyst.

UV-light absorbing groups comprise all groups which absorb light in the range of wavelengths 400 - 320 nm (UVA) and 320 - 290 (UVB) or of even shorter wavelengths (UVC) and which are or can be used as chemical UV filters. These groups are, e.g., residues of compounds belonging to the groups of p-aminobenzoates, salicylates, cinnamates, benzophenones, anthranilates, dibenzoylmethanes, camphor derivatives (such as of benzylidene camphor type), phenyl-benzimidazoles, phenyl-benzoxazoles, phenylbenzotriazoles and others representing state of the art and known to those skilled in the art to be highly active.

The UV-light absorbing groups or compounds mentioned above can be easily modified using methods well-known to a person skilled in the art to yield compounds of formulae VIIIa and IXa above.

Examples of preferred compounds of formulae VIIIa and IXa are: 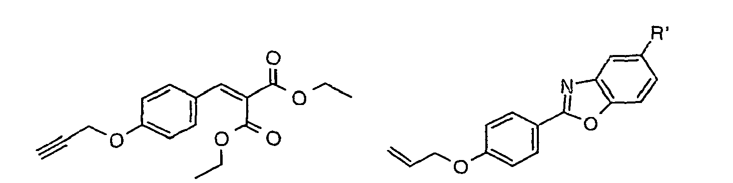 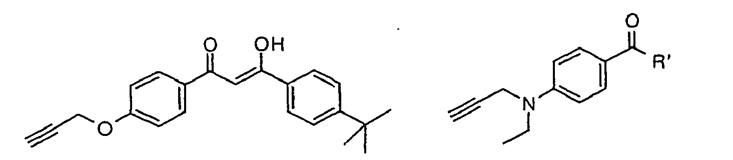 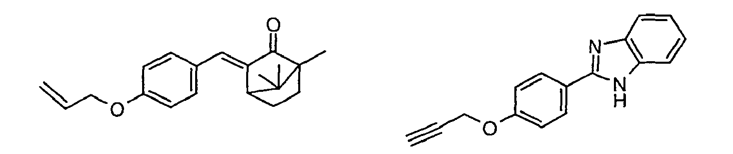 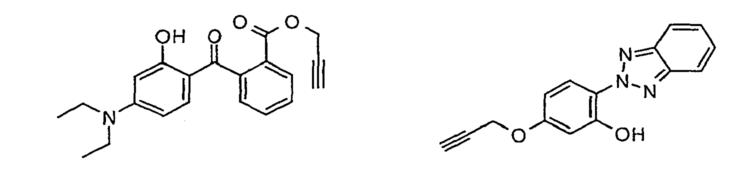 wherein R is hydrogen, hydroxy, straight or branched chain C₁₋₂₀-alkyl, -alkoxy or C₂₋₂₀-alkenyl.

Lipophilic groups are groups with specific affinity to lipids and well-known to persons skilled in the art. Of special interest in the context of the present invention are aromatic, aliphatic and araliphatic hydrocarbon groups. Preferred are straight or branched chain aliphatic hydrocarbon groups with 1 - 20 carbon atoms, one or more isolated methylene groups of which may be replaced by oxygen. Examples of such groups are methyl, ethyl, propyl, i-propyl, butyl, i-butyl, tert.-butyl, pentyl, hexyl, decyl, methoxy, ethoxy, butoxy, methoxymethoxy, ethoxyethoxy, butoxyethoxy, vinyl, alkyl, 2-butenyl, 1,3-butadienyl, etc.

Consequently, examples of compounds of formulae VIIIb and IXb are those wherein R² has one of the above-mentioned meanings, viz., ethylene and acetylene (R² = H), 1-propen, 1 propyne, 1-butene, 1-butyne, 1-pentene, 1-pentyne, 1-heptane, 4-methyl-1-pentene, 3-ethyl-1-pentene, vinyl-methyl-ether, vinyl-ethyl-ether, 1-vinyloxy-methane, 1-vinyloxy-ethane, 1-vinyloxy-butane, 1-(1-vinyloxy)-ethoxy-ethane, etc. Compounds of formulae VIIIb and IXb are either known or can easily be prepared using methods well-known in the art.

The presence of groups R³, i.e. groups able to form ionogenic or hydrogen bonds, in the polysiloxanes of the present invention may be desirable to increase the adhesion of the polysiloxanes to the objects to be protected from the damaging influence of UV radiation, in case of persons skin or hair. Such groups are well-known to the person skilled in the art. Most of these groups are characterized by the existence of free electron pairs. Representatives of such groups are saturated and unsaturated aliphatic and heterocyclic groups having polar substituents with one or more free electron pairs which substituents may be integrated in the carbon chain or ring system. Examples of such substituents are hydroxy, oxo, oxa, carboxy, sulfo, amino, imino and glycyl groups including their salts. Examples of preferred compounds of formulae Xa and Xb are

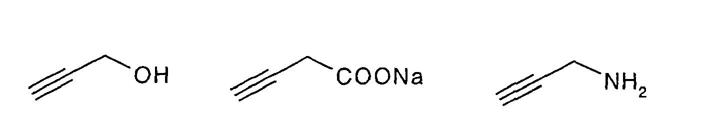

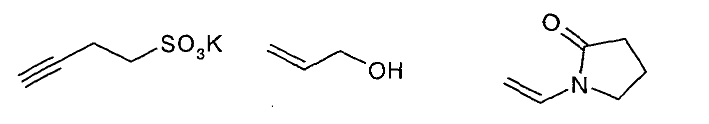

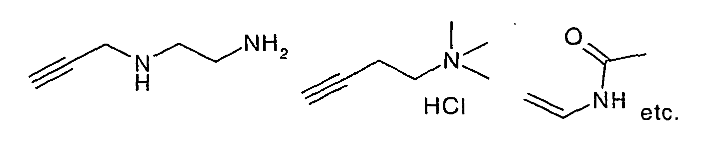

Although not desired, the compounds of the present invention may contain 1 to 20 elements of formula VI, viz. unreacted elements from the starting material. This may be the case when sufficient amounts of reactants of formulae VIII and IX and optionally X were lacking in the reaction mixture or did not react quantitatively for whatever reason. Such compounds are undesired by-products and should be eliminated, since due to the reactivity of the Si-H bond they are not very stable. This can be done, e.g., by a further reaction step. Preferably, elements of formula VI are absent from compounds of the present invention.

The ratio of groups of formula III to formula IV in the compounds of the present invention is not critical. While the preferable mol ratio of the groups is dependent on their molecular weights, the preferable weight ratio of III : IV is about 1:1.

The conditions for the reaction of the polymethylhydrosiloxane of formula VII with the compounds of formulae VIII - X to yield the compounds of the present invention are generally those known to a person skilled in the art.

The reaction is conducted in an organic solvent, e.g., an aliphatic, possibly chlorinated, or aromatic hydrocarbon such as toluene or xylene; an alcohol such as isopropanol; an ether such as THF; or a polar aprotic solvent, such as DMF, which is preferably part of the solvent as a solubilizer.

The reaction temperature, depending upon the reactants, is in the range of 40 to 150°C and preferably about 80°C. The reaction time may vary between 2 and 48 hours.

Preferred noble metal catalysts are platinum metal catalysts, viz. Pt, Pd, Rh and Ru, with platinum being especially preferred. The catalyst can be in heterogeneous phase, e.g., on charcoal or , preferably, in homogeneous phase (Karstedt catalyst).

The reactants are reacted in the desired molecular ratios of the substituents R¹, R² and R³ in the end product either simultaneously or successively under an inert gas atmosphere, preferably under nitrogen or argon. Typically, the polymethylhydrosiloxane is reacted in a first step with a compound of formula VIIIa and optionally of formula Xa carrying the chromophore and optionally substituent R³ in the desired molecular ratio(s), in the presence of an insufficient amount of the lipophilic compound of formula VIIIb and adding an excess of compound VIIIb in a second step. At the end of the reaction time the still existing excess of compound VIIIb is removed. It is evident that the product obtained is a mixture of different polymethylsiloxanes statistically substituted with residues carrying groups R¹ and R² and optionally R³.

Since the compounds of formulae VIII to X can react in 1 - or 2-position (of the terminal double or triple bond) with the polymethylhydrosilane of formula VII a mixture of vicinal (1.2-substitution) and geminal (2.2-substitution) - relative to the terminal double or triple bond of compound VIII to X - reaction products is obtained which can be used as sunscreens without previous separation of its components.

The starting polymethylhydrosiloxane material of formula VII is either commercially available or can easily be prepared by a person skilled in the art from dichloromethylsilane and trimethyl-chlorosilane, using the desired molecular ratios, in aqueous solution.

The polysiloxane compounds of the present invention can be used as sunscreens. They are suitable for the protection of human skin and/or hair from damaging effects of UV radiation, as well as for protection of UV sensitive plastic materials, medicinal products and other objects.

Therefore, the polysiloxane compounds can be converted into compositions, particularly into topical compositions, in combination with pharmaceutically and/or cosmetically acceptable excipients.

The compositions comprising the compounds of the present invention are particularly suitable for topical applications onto human skin and/or hair.

If desired, additional UV-A and UV-B screening agents may be added to the cosmetic and/or dermatological compositions of the present invention. The combination of different UV filters may also show synergistic effects.

The total amount of UV screening agents, i.e. of the present compounds and of additional UV-A/B screening agents, is not critical. Suitable amounts may vary between 0.5 and 20%, preferably between 0.5 and 12%, by weight of the total amount of the composition.

Suitable UV-B screening agents which may be contained in the compositions of the present invention are, e.g., the following organic and inorganic compounds:
Acrylates, such as 2-ethylhexyl-2-cyano-3,3-diphenylacrylate (octocrylene, PARSOL® 340), ethyl 2-cyano-3,3-diphenylacrylate and the like;
camphor derivatives such as 4-methyl benzylidene camphor (PARSOL® 5000), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid and the like;
cinnamate derivatives such as octyl methoxycinnamate (PARSOL® MCX), ethoxyethyl methoxycinnamate, diethanolamine methoxycinnamate (PARSOL® Hydro), isoamyl methoxycinnamate and the like, as well as cinnamic acid derivatives bound to siloxanes;
p-aminobenzoic acid derivatives, such as p-aminobenzoic acid, 2-ethylhexyl p-dimethylaminobenzoate, N-oxypropylenated ethyl p-amonibenzoate, glyceryl p-aminobenzoate;
benzophenones, such as benzophenone-3, benzophenone-4, 2, 2', 4, 4'-tetrahydroxybenzophenone, 2,2'-dihydroxy-4,4'-dimethoxybenzophenone and the like;
esters of benzalmalonic acid such as di-(2-ethylhexyl)-4-methoxybenzalmalonate;
esters of 2-(4-ethoxy-anilinomethylene)-propandioic acid, such as 2-(4-ethoxy-anilinomethylene)-propandioic acid diethyl ester (EP-A2- 0895 776);
organosiloxane compounds containing benzmalonate groups as described in the European Patent Publications EP-B1-0358584, EP-B1-0538431 and EP-A1-0709080;
drometrizole trisiloxane (Mexoryl XL);
pigments such as microparticulated TiO₂, and the like, the term "microparticulated" referring to a particle size from about 5 nm to about 200 nm, particularly from about 15 nm to about 100 nm. The TiO₂ particles may also be coated by metal oxides such as aluminum or zirconium oxide, or by organic coatings such as polyols, methicone, aluminum stearate, alkyl silane and the like, well known in the art;
imidazole derivatives such as, 2-phenyl benzimidazole sulfonic acid and its salts (PARSOL®HS), e.g., alkali salts such as sodium or potassium salts, ammonium salts, morpholine salts, salts of primary, sec. and tert. amines like monoethanolamine salts, diethanolamine salts and the like;
salicylate derivatives such as isopropylbenzyl salicylate, benzyl salicylate, butyl salicylate, octyl salicylate (NEO HELIOPAN OS), isooctyl salicylate or homomenthyl salicylate (homosalate, HELIOPAN) and the like;
triazone derivatives such as octyl triazone (UVINUL T-150), dioctyl butamido triazone (UVASORB HEB) and the like.

Suitable conventional UV-A screening agents which may be contained in the compositions of the present invention are the following organic and inorganic compounds:
Dibenzoylmethane derivatives such as 4-tert.-butyl-4'-methoxydibenzoyl-methane (PARSOL® 1789), dimethoxydibenzoylmethane, isopropyldibenzoylmethane and the like;
benzotriazole derivatives such as 2,2'-methylene-bis-[6-(2H-benzotriazole-2-yl)-4-(1,1,3,3,-tetramethylbutyl)-phenol] (TINOSORB M) and the like;
phenylene-1,4-bis-benzimidazolsulfonic acids or their salts such as 2,2-(1,4-phenylene)-bis-(1H-benzimidazol-4,6-disulfonic acid) (Neoheliopan AP);
amino substituted hydroxybenzophenones such as 2-(4-diethylamino-2-hydroxybenzoyl)-benzoic acid hexylester as described in European patent publication EP 1046391;
pigments such as microparticulated ZnO and the like. The term "microparticulated" refers to a particle size from about 5 nm to about 200 nm, particularly from about 15 nm to about 100 nm. The ZnO particles may also be coated by metal oxides such as, e.g., aluminum or zirconium oxides or by organic coatings such as e.g. polyols, methicone, aluminum stearate, alkyl silane. Such coatings are well known in the art.

Because dibenzoylmethane derivatives are photolabile UV-A screening agents, it may be desirable to photostabilize them. Thus, the term "conventional UV-A screening agent" also refers to dibenzoylmethane derivatives such as e.g. PARSOL® 1789 stabilized by, e.g.,
3,3-diphenylacrylate derivatives as described in EP-B1-0514491 and EP-A1-0780119;
benzylidene camphor derivatives as described in USP-5605680;
organosiloxanes containing benzmalonate groups as described in EP-B1-0358584, EP-B1-053843 and EP-A1-0709080.

The compositions of the invention can also contain usual cosmetic adjuvants and additives, such as preservatives/ antioxidants, fatty substances/ oils, water, organic solvents, silicones, thickeners, softeners, emulsifiers, additional sunscreens, antifoaming agents, moisturizers, fragrances, surfactants, fillers, sequestering agents, anionic, cationic, nonionic or amphoteric polymers or mixtures thereof, propellants, acidifying or basifying agents, dyes, colorants, pigments or nanopigments, in particular those suitable for providing an additional photoprotective effect by physically blocking out ultraviolet radiation, or any other ingredients usually formulated into cosmetics, in particular for the production of sunscreen/antisun compositions. The necessary-amounts of the cosmetic and dermatological adjuvants and additives can, based on the desired product, easily be chosen by the skilled person.

Particularly preferred antioxidants are those chosen from the group consisting of amino acids (e.g. glycine, histidine, tyrosine, tryptophane) and their derivatives, imidazole (e.g urocanic acid) and derivatives, peptides such as D,L-carnosine, D-carnosine, L-carnosine and derivatives (e.g. anserine), carotinoids, carotenes (e.g. β-carotene, γ-carotene, lycopene) and derivatives, chlorogenic acid and derivatives, liponic acid and derivatives (e.g. dihydroliponic acid), aurothioglucose, propylthiouracil and other thiols (e.g. thioredoxine, glutathione, cysteine, cystine, cystamine and its glycosyl-, N-acetyl-, methyl-, ethyl-, propyl-, amyl-, butyl- and lauryl-, palmitoyl-, oleyl-, γ-linoleyl-, cholesteryl- and glycerylester) and the salts thereof, dilaurylthiodipropionate, distearylthiodipropionate, thiodipropionic acid and its derivatives (esters, ethers, peptides, lipids, nucleotides, nucleosides and salts) as well as sulfoximine compounds (such as buthioninsulfoximine, homocysteinsulfoximine, buthioninsulfone, penta-, hexa-, heptathioninsulfoximine) in very low compatible doses (e.g. from pmol to µmol/kg), additional (metal)-chelators (such as α-hydroxyfatty acids, palmic acid, phytinic acid, lactoferrin), α -hydroxyacids (such as citric acid, lactic acid, malic acid), huminic acid, gallic acid, gallic extracts, bilirubin, biliverdin, EDTA, EGTA and its derivatives, unsaturated fatty acids and their derivatives (such as γ-linoleic acid, linolic acid, oleic acid), folic acid and its derivatives, ubiquinone and ubiquinol and their derivatives, vitamine C and derivatives (such as ascorbylpalmitate, Mg-ascorbylphosphate, Na-ascorbylphosphate, ascorbylacetate), tocopherole and derivates (such as vitamine E acetate), vitamine A and derivatives (vitamine A palmitate) as well as coniferylbenzoat, rutinic acid and derivatives, α-glycosylrutin, ferulic acid, furfurylidenglucitol, carnosin, butylhydroxytoluene, butylhydroxyanisole, trihydroxybutyrophenone, urea and its derivatives; mannose and derivatives, zinc and deivatives (e.g. ZnO; ZnS0₄), Selen and derivatives (e.g. selenomethionin), stilbenes and derivatives (such as stilbenoxide, transstilbenoxide) and suitable derivatives (salts, esters, ethers, sugars, nucleotides, nucleosides, peptides and lipids) of the named active ingredients.

The preservatives and/or antioxidants may be present in an amount varying from about 0.01 wt.% to about 10 wt.% of the total weight of the composition. Preferably, the preservatives and/or antioxidants are present in an amount varying from about 0.1 wt.% to about 1 wt.%.

The compositions according to the present invention may also contain emulsifiers. An emulsifier enables two or more immiscible liquids to be combined homogeneously, while increasing the viscosity of the composition. Moreover, the emulsifier acts to stabilize the composition.

Emulsifiers that may be used according to the present invention, to form O/W, W/O and/or O/W/O formulations, include sorbitan oleate, sorbitan sesquioleate, sorbitan isostearate, sorbitan trioleate, polyglyceryl-3-diisostearate, polyglycerol esters of oleic/isostearic acid, polyglyceryl-6 hexaricinolate, polyglyceryl-4-oleate, polygylceryl-4 oleate/PEG-8 propylene glycol cocoate, oleamide DEA, TEA myristate, TEA stearate, magnesium stearate, sodium stearate, potassium laurate, potassium ricinoleate, sodium cocoate, sodium tallowate, potassium castorate, sodium oleate, and mixtures thereof. Further suitable emulsifiers are phosphate esters and salts thereof such as cetyl phosphate, DEA cetyl phosphate, potassium cetyl phosphate, sodium glyceryl oleate phosphate, hydrogenated vegetable glyceride phosphates and mixtures thereof. Furthermore, one or more synthetic polymers may be used as emulsifiers. For example, PVP eicosaene copolymer, acrylates/C10-C30 alkyl acrylate crosspolymer, acrylates/steareth methacrylate copolymer, PEG-22/dodecyl glycol copolymer, PEG-45/dodecyl glycol copolymer, and mixtures thereof. Preferred emulsifiers are PVP eicosaene copolymer, acrylates/C10-C30 alkyl acrylate crosspolymer, PEG-20 sorbitan isostearate, sorbitan isostearate, and mixtures thereof.

The emulsifier may be present in a total amount varying from about 0.01 wt.% to about 15 wt.%, preferably from about 0.1 wt.% to about 3 wt.%, of the total weight of the composition.

The fatty/oily phase is advantageously chosen from:
mineral oils and mineral waxes;
oils such as triglycerides of caprinic acid or caprylic acid, preferably castor oil;
natural or synthetic oils, preferably esters of carbonic acids or fatty acids with alcohols, e.g., such as isopropanol, propyleneglycol or glycerine;
alkylbenzoates and
silicone oils such as dimethylpolysiloxane, diethylpolysiloxane, diphenylpolysiloxane and mixtures thereof.

Fatty substances which can be incorporated into the oily phase of the compositions according to the invention are advantageously chosen from esters of saturated and/or unsaturated, straight or branched chain alkyl carboxylic acids with 3 to 30 carbon atoms, and saturated and/or unsaturated, straight and/or branched chain alcohols with 3 to 30 carbon atoms, as well as esters of aromatic carboxylic acids and of saturated and/or unsaturated, straight or branched chain alcohols of 3 to 30 carbon atoms. Such esters can advantageously be selected from octylpalmitate, octylcocoate, octylisostearate, octyldodecylmyristate, cetylisononanoate, isopropylmyristate, isopropylpalmitate, isopropylstearate, isopropyloleate, n-butylstearate, n-hexyllaurate, n-decyloleat, isooctylstearate, isononylstearate, isononylisononanoate, 2- ethyl hexylpalmitate, 2-ethylhexyllaurate, 2-hexyldecylstearate, 2-octyldodecylpalmitate, stearylheptanoate, oleyloleate, oleylerucate, erucyloleate, erucylerucate, tridecylstearate, tridecyltrimellitate, as well as from synthetic, half-synthetic and natural mixtures of such esters such as jojoba oil.

Other fatty components suitable for use in the compositions according to the present invention include polar oils such as lecithines and fatty acid triglycerides, namely triglycerinic esters of saturated and/or unsaturated, straight or branched chain carbonic acids with 8 to 24 carbon atoms, preferably of 12 to 18 carbon atoms whereas the fatty acid triglycerides are preferably chosen from synthetic, half synthetic and natural oils (e.g. cocoglyceride, olive oil, sun flower oil, soybean oil, peanut oil, rape oil, almond oil, palm oil, coconut oil, castor oil, hydrogenated castor oil, wheat oil, grape oil and others); apolar oils such as linear and/ or branched chain hydrocarbons and waxes, e.g., mineral oils, vaseline (petrolatum); paraffins, squalan and squalen, polyolefines (favored are polydecenes), hydrogenated polyisobutenes and isohexadecanes; dialkyl ethers such as dicaprylylether; linear or cyclic silicone oils such as cyclomethicone, octamethylcyclotetrasiloxane, cetyldimethicone, hexamethylcyclotrisiloxane, polydimethylsiloxane, poly-(methylphenylsiloxan) and mixtures thereof.

Other fatty components which can advantageously be incorporated into the compositions of the present invention are isoeikosane; neopentylglycoldiheptanoate; propylenglykoldicaprylate/-dicaprate; caprylic-/capric-/diglycerylsuccinate; butylenglykol caprylat/caprat; C12-13 alkyllactate; di-C12-13 alkyltartrate; triisostearin; dipentaerythrityl hexacaprylat/hexacaprate; propylenglykolmonoisostearate; tricaprylin; dimethylisosorbid. Particularly preferred is the use of mixtures of C12-15 alkylbenzoate and 2-ethylhexylisostearate, mixtures of C12-15 alkylbenzoate and isotridecylisononanoate as well as mixtures of C12-15 alkylbenzoate, 2-ethylhexylisostearate and isotridecylisononanoate.

The oily phase of the compositions according to the present invention can also contain natural vegetable or animal waxes such as bee wax, china wax, bumblebee wax and other waxes of insects as well as sheabutter.

The compositions according to the present invention may additionally contain one or more emollients. An emollient provides a softening or soothing effect on the skin surface and is generally considered safe for topical use. Emollients also help control the rate of evaporation and the tackiness of the composition. Preferred emollients include mineral oil, lanolin oil, coconut oil, cocoa butter, olive oil, aloe extracts, jojoba oil, castor oil, fatty acids such as oleic and stearic acid, fatty alcohols such as cetyl and hexadecyl alcohol diisopropyl adipate, benzoic and hydroxybenzoic acid esters of C₉-C₁₅ alcohols, isononyl iso-nonanoate, C₁₅-C₅₀ alkanes, mineral oil, silicones such as dimethyl polysiloxane, ethers such as polyoxypropylene butyl ethers and polyoxypropylene cetyl ethers, and C₂-C₁₅ alkyl benzoates, and mixtures thereof. The most preferred emollients are hydroxybenzoate esters, aloe vera, C₁₂₋₁₅ alkyl benzoates, and mixtures thereof.

The emollient is present in an amount varying from about 1 wt.% to about 20 wt.%, preferably from about 2 wt.% to about 15 wt.%, and most preferrably from about 4 wt.% to about 10 wt.% of the total weight of the composition.

The aqueous phase of the formulations of the present invention can contain the usual cosmetic additives such as alcohols, especially lower alcohols, preferably ethanol and/ or isopropanol, low alkyl diols or polyols and their ethers, preferably propyleneglycol, glycerine, ethyleneglycol, ethyleneglycolmonoethyl- or -monobutyl ether, propyleneglycolmonomethyl-, -monoethyl- or -monobutyl ether, diethyleneglycolmonomethyl- or -monoethyl ether and analogue products, polymers, foam stabilisators; electrolytes and, especially, one or more thickeners.

Thickeners that may be used in formulations of the present invention include the family of siliciumdioxide, magnesium and/or aluminum silicates, polysaccharides and their derivatives such as hyaluronic acid, xanthan gum, hydroxypropyl cellulose, acrylate copolymers, preferably a polyacrylate of the family of carbopoles, such as carbopoles of type 980, 981, 1382, 2984, 5984.

Moisterizing agents, such as humectants, may be incorporated into the compositions according to the present invention to reduce the trans-epidermal water loss (TEWL) of the horny layer of the skin. Suitable humectants include glycerin, lactic acid, pyrrolidone carbonic acid, urea, polyethylene glycol, polypropylene glycol, sorbitol, PEG-4, and mixtures thereof. Additional suitable moisturizers are polymeric moisturizers of the familiy of water soluble and/or with water gelating polysaccarides such as hyaluronic acid, chitosan and/or fucose rich polysaccharides available, e.g., as Fucogel®1000 (CAS-Nr. 178463-23-5) from SOLABIA S. The moisterizing agent is optionally present in an amount varying from about 0.5 wt.% to about 8 wt.%, preferably from about 1 wt.% to about 5 wt.% of the total weight of the composition.

Suitable neutralizing agents which may be included in the composition of the present invention to neutralize components such as e.g. an emulsifier or a foam builder/stabilizer include but are not limited to alkali hydroxides such as a sodium and potassium hydroxide; organic bases such as diethanolamine (DEA), triethanolamine (TEA), aminomethyl propanol, trisodium ethylenediaminetetraacetic acid and mixtures thereof; basic amino acids such as arginine and lysine and any combination of any of the foregoing. The neutralizing agent may be present in an amount of about 0.01 wt.% to about 8 wt.% in the compositions of the present invention, preferably, 1 wt.% to about 5 wt.%.

The addition of electrolytes into the composition of the present invention may be necessary to change the behavior of a hydrophobic emulsifier. Thus the emulsions/ microemulsions of this invention may preferably contain electrolytes of one or several salts including anions such as a chloride, a sulfate, a carbonate, a borate or an aluminate, without being limited thereto. Other suitable electrolytes may be on the bases of organic anions such as, but not limited to, lactate, acetate, benzoate, propionate, tartrate and citrate. As cations preferably ammonia, alkylammonia, alkali- or alkaline earth metals, magnesium-, iron- or zinc-ions are selected. Especially preferred salts are potassium and sodium chloride, magnesium sulfate, zinc sulfate and mixtures thereof. Electrolytes are present in an amount of about 0.01 wt.% to about 8 wt.% in the compositions of the present invention.

The cosmetic compositions of the invention are useful as compositions for photoprotecting the human epidermis or hair against the damaging effect of UV irradiation, as antisun/ sunscreen composition or as makeup product. Such compositions can, in particular, be provided in the form of a lotion, a thickened lotion, a gel, a cream, a milk, an ointment, a powder or a solid tube stick and may optionally be packaged as an aerosol and may be provided in the form of a mousse, foam or a spray. When the cosmetic composition according to the invention is provided for protecting the human epidermis against UV radiation or as antisun/ sunscreen composition, it may be in the form of a suspension or dispersion in solvents or fatty substances, or alternatively in the form of an emulsion or microemulsion (in particular of O/W or W/O type, O/W/O or W/O/W-type), such as a cream or a milk, a vesicular dispersion, in the form of an ointment, a gel, a solid tube stick or an aerosol mousse. The emulsions can also contain anionic, nonionic, cationic or amphoteric surfactants.

When the cosmetic composition according to the invention is used for protecting the hair, it may be in the form of a shampoo, a lotion, a gel or a rinse out composition, to be applied before or after shampooing, before or after dyeing or bleaching, before, during or after permanent-waving or hair straightening operation, a styling or treatment lotion or a gel, a blow-drying or hairsetting lotion or gel, a hair lacquer, or a composition for permanent-waving, straightening, dyeing or bleaching the hair.

When the cosmetic composition according to the invention is used as makeup product for eyelashes, the eyebrows, the skin or the hair, such as an epidermal treatment cream, a foundation, a tube oflipstick, an eyeshadow, a face powder, an eyeliner, a mascara or a coloring gel, it may be solid or pasty, anhydrous or in aqueous form, such as O/W or W/O emulsion, suspension or gel.

The present invention also features formulating the polysiloxane compounds according to the invention as agents for screening out UV radiation, in particular for controlling the color of human skin.

The polysiloxane compounds according to this invention show an excellent liposolubility and can thus be incorporated in high concentrations into cosmetic formulations leading to a high protection factor of the final compositions. Additionally they are homogeneously distributed in the cosmetic formulation containing at least a fatty phase and a cosmetically accepted organic solvent which leads, applied on the skin/ or hair, to the formation of a protective film which protects effectively the skin and/ or hair against the deleterious effects of UV-radiation.

Thus, it is another object of the present invention to use the compounds and compositions of the invention for protecting the skin and/ or hair against UV radiation, in particular solar radiation, comprising topically applying an effective amount of a cosmetic composition containing the polysiloxane compounds according to the invention.

Finally, according to another embodiment of the invention, a polysiloxane compound or compositions of this invention can be used as protecting agents against UV radiation for plastics and other UV sensitive materials and products.
The following Examples are illustrative of the present invention without limiting it.

### Example 1

Polysiloxane copolymer containing groups of formula III c wherein R¹ is 4-(2-benzoxazolyl)-phenoxymethyl and of formula IV b wherein R² is butyloxy.

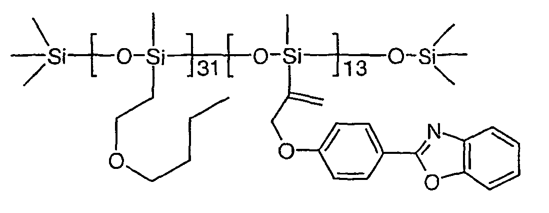

### a) 4-(2-Benzoxazolyl)-phenol

A 250 ml three necked reaction flask, equipped with a reflux condenser combined with a water separator and an oil bath with a magnetic stirrer was charged with 16.4g (150mmol) of 2-aminophenol (Fluka), 20.8g (150 mmol) of 4-hydroxy-benzoic acid and 1.2g of boric acid in 150 ml of 1,2-dichlorobenzene. After 18 hours of reflux 5 ml of water were separated. On tlc (hexane / ethylacetate = 3:2) no starting material could be observed. The reaction mixture was cooled and diluted with hexane. Crystals were formed, which were filtered off, washed with hexane and dried at 60°C in a vacuum to yield 31.3g (99%) of the product. M.p. 242-245°C. UV(ethanol) 306 nm (31'831); MS: 210 (M-H).

### b) 2-(4-Prop-2-ynyloxy-phenyl)-benzoxazole

A 1000 ml round bottom flask, equipped with a reflux condenser and an oil bath with a magnetic stirrer was charged with 21.1g of the above 4-(2-benzoxazolyl)-phenol, 14.3g of propargyl bromide and 26.5g of anhydrous Na₂CO₃ in 500 ml of 1-methyl-2-pyrrolidone. A trace of KJ was added. After one hour at 110°C no starting material could be observed on tlc. The reaction mixture was diluted with ethylacetate and washed with In NaOH, water and NaCl solution, dried with Na₂SO₄, concentrated and recrystallised from toluene to yield 17.3g (70%) of 2-(4-prop-2-ynyloxy-phenyl)-benzoxazole. M.p. 124-127°C; UV(ethanol) 306 nm (30'691).

### c) Polysiloxane copolymer

A 50 ml round bottom flask, equipped with a reflux condenser and an oil bath with a magnetic stirrer was charged with 2g of polymethylhydrosiloxane (PS-118 of United Chemical Technol. Inc., CAS 63148-57-2), 2.3g (0.3 Mol equiv.) of the above 2-(4-prop-2-ynyloxy-phenyl)-benzoxazole and 2.13g (0.7 Mol equiv.) of n-butyl-vinylether in 20 ml of toluene under nitrogen atmosphere. The reaction mixture was heated to 80°C and a catalytic amount of divinyl-tetramethyl disiloxane platinum complex was added. The reaction mixture was heated for 18 hours, until no Si-H bond could be detected in the IR spectrum at 2120 cm⁻¹ anymore. The product solution was treated with charcoal and concentrated to yield 4.4g (70% of the theory) of a slightly yellowish, honey like liquid. UV 302 nm (E=561). Its NMR shows a mixture of the vicinal (formula III d) and the geminal (formula III c) hydrosilylation product = 1 : 3. It is easily miscible with cosmetic solvents and has excellent photostability qualities measured in a film according to the method of G. Berset et. al., International Journal of Cosmetic Science 18, 167-177 (1996).

### Example 2

Polysiloxane copolymer containing groups of formula III b wherein R¹ is 4-(2-benzoaxzolyl)- phenoxymethyl and of formula IV b wherein R² is butyloxy.

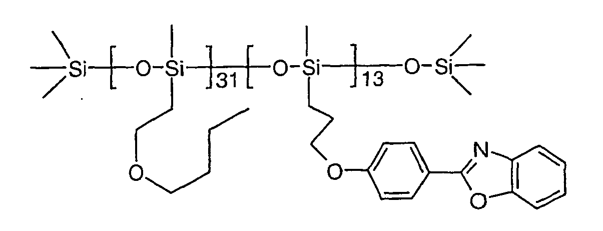

### a) 2-(4-prop-2-enyloxy-phenyl)-benzoxazole

The same reaction as in Example 1b was performed, but instead of propargyl bromide, allyl bromide was used. After workup, reddish crystals were obtained, which were recrystallized from hexane (yield 93% of the theory). M.p. 95-98°C; UV (EtOH) 306 nm (34'085 / E=1358).

### b) Polysiloxane copolymer

The same reaction conditions as.in Example 1c were used, but instead of 2-(4-prop-2-ynyloxy-phenyl)-benzoxazole, 2.28 g of the above 2-(4-prop-2-enyloxy-phenyl)-benzoxazole were added. After 20 hours at 80°C and work up as described above, 4.59g of the product as clear, brownish oil were obtained. UV (EtOH) 302 nm (E=573).

### Example 3

Polysiloxane copolymer containing groups of formula III c wherein R¹ is 4-[(2,2-diethoxycarbonyl)-vinyl]- phenoxymethyl and of formula IV b wherein R² is butyloxy.

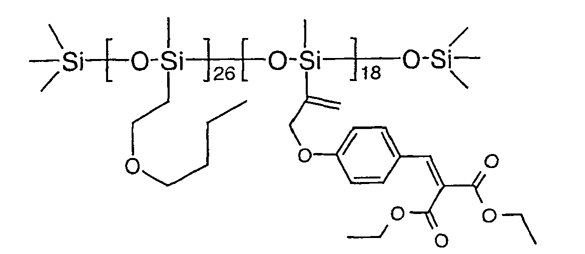

The same reaction conditions as in Example 1c were used, but instead of 2-(4-prop-2-ynyloxy-phenyl)-benzoxazole, 2.75 g of 2-(4-prop-2-ynyloxy-benzylidene)-malonic acid diethyl ester (prepared according to G. Frater et.al., EP 05380 431 [1991], Example 1) were added. After 18 hours at 80°C and work up as described above, 4.98g (71%) of the product as a yellowish honey were obtained. UV (EtOH) 310 nm (E=402).

### Example 4

Polysiloxane copolymer containing groups of formula III c wherein R¹ is 4-[(2,2-diethoxycarbonyl)-vinyl]-phenoxymethyl and of formula IV b wherein R² is hydrogen.

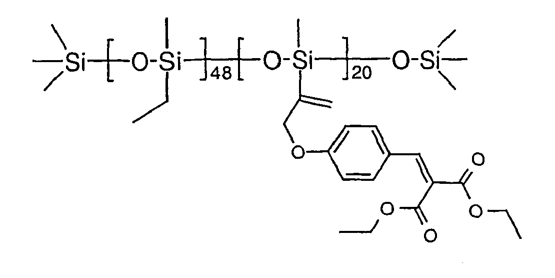

A 150 ml autoclave with a glass insertion was charged with 5g of polymethyl-hydrosiloxane (HMS-991 of ABCR, D-76189, Karlsruhe, viscosity 15-25 cSt., CAS 63148-57-2), 2.75 g of 2-(4-prop-2-ynyloxy-benzylidene)-malonic acid diethyl ester (prepared according to G. Frater et al., EP 05380 431 [1991]) and four drops of divinyl-tetramethyl disiloxane platinum complex (Degussa Nr. 19031410% in toluene) in 25 ml of toluene under nitrogene atmosphere. The autoclave was closed and an excess of ethylene gas was introduced in the apparatus with some pressure. The reaction mixture was heated to 50°C for 22 hours. Then the temperature was set to 80°C for six additional hours. In a sample no Si-H bond could be detected in the IR spectrum at 2150 cm⁻¹ anymore. The product solution was treated with charcoal and concentrated to yield 13.75g of a slightly orange oil. UV (EtOH) 310 nm (E=389).

### Example 5

Polysiloxane copolymer containing groups of formula III c wherein R¹ is 4-[5-(1,1-dimethyl-propyl)-2-benzoxazolyl]-phenoxymethyl and of formula IV b wherein R² is hydrogen.

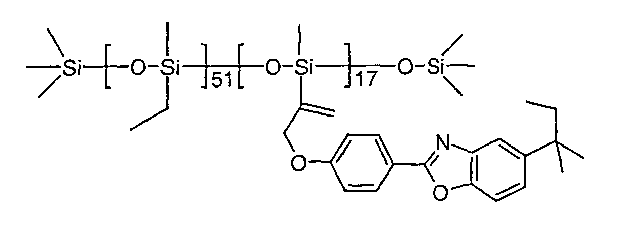

### a) 2-Amino-4-(1,1-dimethyl-propyl)-phenol

A 250 ml round bottom flask, equipped with a magnetic stirrer and an ice bath under nitrogen atmosphere was charged with 10.9g of 2-aminophenol and 13g of 2-cloro-2-methylbutane. 50 ml of conc. sulfuric acid was slowly added under stirring and cooling so that the temperature was never exceeding 0°C. 2g of Nafion^{®}-H was added and the mixture was stirred for one hour. On tlc no starting material could then be observed. The reaction mixture was thrown on ice and neutralized to pH 8 with Na₂CO₃. The product was extracted with ethyl acetate, the organic phases were dried with Na₂SO₄ and concentrated and recrystallized from toluene to yield 11.5g of off white crystals. M.p. 116-119°C; MS: 179 (M⁺), 150 (100%).

### b) 4-[5-(1,1-Dimethyl-propyl)-benzoxazole-2-yl]-phenol

The reaction of Example 1a was repeated, using the above 2-amino-4-(1,1-dimethylpropyl)-phenol instead of 2-aminophenol. After reflux over night, the reaction mixture was cooled, diluted with hexane and filtered off, to yield 85% of brown crystals. M.p. 232-235°C; UV (EtOH) 310 nm (32'320). According to NMR, about 7% of 4-[5-tert.-butyl-2-benzoxazolyl]-phenol homologue was observed.

### c) 5-(1,1-Dimethyl-propyl)-2-(4-prop-2-ynyloxy-phenyl)-benzoxazole

The reaction of Examplelb was repeated using the above 4-[5-(1,1-dimethyl-propyl)-benzoxazole-2-yl]-phenol instead of 4-(2-benzoxazolyl)-phenol. A brown crystalline product was obtained in 94% yield. M.p. 61-64°C; UV (EtOH) 310 nm (32'310), MS: 319 (M⁺), 304, 290 (100%), 251.

### d) Polysiloxane copolymer

The same reaction conditions as in Example 4 were used, but instead of 2-(4-prop-2-ynyloxy-benzylidene)-malonic acid diethyl ester, 2.28 g (25 Mol equiv.) of the above 5-(1,1-dimethyl-propyl)-2-(4-prop-2-ynyloxy-phenyl)-benzoxazole were added to 2g of HMS-991 (ABCR) in 20 ml of toluene and 2 drops of the catalyst. 5.5g of a honey like product were obtained. UV (EtOH) 312 nm (E=436).

### Example 6

Polysiloxane copolymer containing groups of formula III c wherein R¹ is 4-[(2,2-diethoxycarbonyl)-vinyl]- phenoxymethyl and of formula IV b wherein R² is butyl.

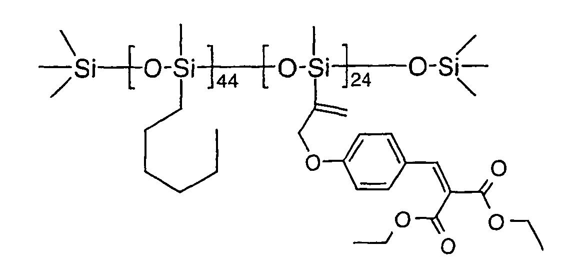

A 25 ml round bottom flask, equipped with a reflux condenser and an oil bath with a magnetic stirrer was charged with 1g (1 Mol equiv.) of polymethylhydrosiloxane (HMS-991 of ABCR, D-76189, Karlsruhe, viscosity 15-25 cSt., CAS 63148-57-2), 1.5 g (0.3 Mol equiv.) of 2-(4-prop-2-ynyloxy-benzylidene)-malonic acid diethyl ester (prepared according to G. Frater et al., EP 05380 431 [1991]) and 1.08g (0.8 Mol equiv.) of 1-hexene in 6 ml of toluene under nitrogen atmosphere. The reaction mixture was heated to 55°C and two drops of divinyl-tetramethyl disiloxane platinum complex was added. The reaction mixture was heated for 25 hours, until no Si-H bond could be detected in the IR spectrum at 2120 cm⁻¹ anymore. The product solution was treated with charcoal and concentrated to yield 2.1g of a clear honey like liquid. UV 310 nm (E=343). It is easily miscible with cosmetic solvents and has excellent photostability qualities measured in a film according to the method of G. Berset et al., International Journal of Cosmetic Science 18, 167-177 (1996).

### Example 7

Polysiloxane copolymer containing groups of formula III c wherein R¹ is 4-[(2,2-diethoxycarbonyl)-vinyl]- phenoxymethyl and of formula IV b wherein R² is isobutyl.

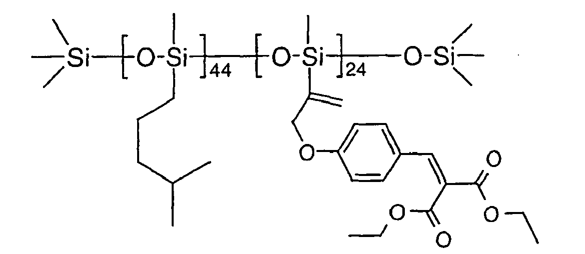

A similar result was obtained as in Example 6, with 1.08g of 4-methyl-1-pentene used instead of 1-hexene.

### Example 8

UV-A/B sunscreen: Polysiloxane copolymer containing groups of formula III c wherein R¹ is 4-[(2,2-diethoxycarbonyl)-vinyl]- phenoxymethyl, formula III c wherein R¹ is 2-(4-diethylamino-2-hydroxybenzoyl)-benzoyloxymethyl and of formula IV b wherein R² is butyloxy.

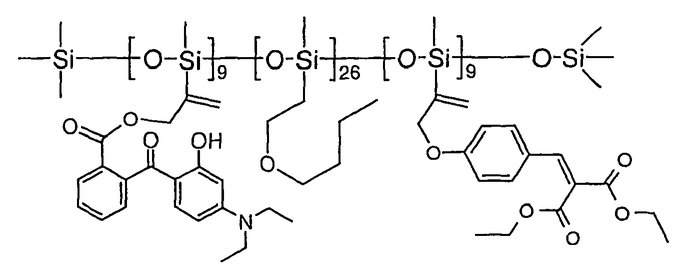

### a) Preparation of 2-(4-diethylamino-2-hydroxybenzoyl)-benzoic acid prop-3-ynyl ester

A mixture of 32 mmol of 2-(4-diethylamino-2-hydroxybenzoyl)-benzoic acid, 160 mmol of propargyl alcohol and 32 mmol of H₂SO₄ is refluxed for 48 hours. After evaporation of the excess propargyl alcohol the residue is dissolved in ethylacetate and subsequently washed twice with saturated NaHCO₃ solution and twice with water. After drying with Na₂SO₄, the solvent is evaporated under high vacuum and the crude product is purified twice chromatographically (hexane/EtOAc 1:1, hexane/methyl-tert.-butyl-ether 4 : 1-1 : 1) yielding 2-(4-diethylamino-2-hydroxybenzoyl)-benzoic acid prop-2-ynyl ester. MS (EI): 374 (18%, M+Na⁺), 352 (100%, M+ H⁺), 296 (17%). UV (EtOH): λₘₐₓ= 356 nm (ε = 31'648).

### b) Polysiloxane copolymer

The same reaction conditions as in Example 1c are used, but instead of 2-(4-prop-2-ynyloxy-phenyl)-benzoxazole, 1.4 g of 2-(4-prop-2-ynyloxy-benzylidene)-malonic acid diethyl ester (prepared according to G. Frater et al., EP 05380 431 [1991], Example 1) and 1.6g of 2-(4-diethylamino-2-hydroxybenzoyl)-benzoic acid prop-2-ynyl ester are added. After 18 hours at 80°C and work up as described above, 5g of the product as a yellowish honey is obtained. UV (EtOH) 310 nm (E=200) and 356 nm (E=202).

### Example 9

Polysiloxane copolymer containing groups of formula III c wherein R¹ is 4-[(2,2-diethoxycarbonyl)-vinyl]- phenoxymethyl, of formula Vb wherein R³ is 2-oxopyrrolidino and of formula IV b wherein R² is butyloxy.

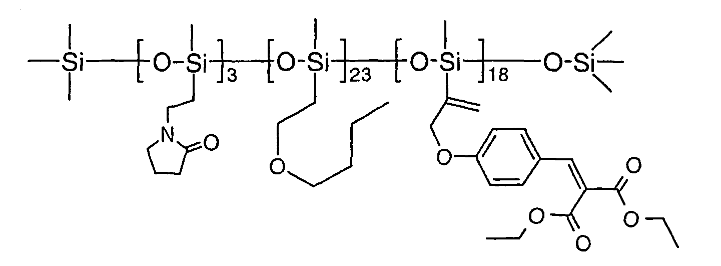

The same reaction conditions as in Example 1c are used, but instead of 2-(4-prop-2-ynyloxy-phenyl)-benzoxazole, 2.75 g of 2-(4-prop-2-ynyloay-benzylidene)-malonic acid diethyl ester (prepared according to G. Frater et.al., EP 05380 431 [1991], Example 1), 1.8g (0.6 Mol equiv.) of n-butyl-vinylether and 0.23g of 1-vinyl-2-pyrrolidone are added. After 8 hours at 30°C the same amount of n-buryl-vinylether is added again and heated for further 18 hours. After work up as described above, 5g of the product as a yellowish honey is obtained. UV (EtOH) 310 nm (E=400).

### Example 11

### Preparation of a O/W sunscreen lotion UV-B and UV-A:

Broad spectrum sunscreen lotion containing 2% of the compound of Example 1.

| Recipe % | **Compound** | **Chemical Name** |
|---|---|---|
| **Part A** | | |
| 2 | PARSOL MCX | Ethylhexyl methoxycinnamate |
| 2 | Product of Example 1 | |
| 3 | PARSOL 1789 | 4-t-Butyl-4'-methoxy-dibenzoyl-methane |
| 12 | Cétiol LC | Cocoyl-caprylate caprate |
| 4 | Dermol 185 | Isostearyl neopentanoate |
| 0.25 | PEG-2-stearate | Diethyleneglycol monostearate |
| 1 | Cetylalcohol | Cetylalcohol |
| 0.25 | MPOB/PPOB | Methyl-propylparabene |
| 0.1 | EDTA BD | EDTA-sodium salt |
| 1 | Amphisol DEA (Givaudan) | Diethanolamine cetylphosphate |

| **Part B** | | |
|---|---|---|
| 0.2 | Permulene TR-1 | Acrylate C10-C30 alkylacrylate |
| 68.4 | Water deionized | Water deionized |
| 5 | Propyleneglycol | 1,2-Propanediol |
| 0.8 | KOH(10%) | Potassium hydroxide |

Part A is heated in a reactor to 85°C. Part B is slowly added within 10 minutes, followed by addition of KOH, cooling and degassing of the emulsion.

### Example 11

### Sun milk waterproofed

| %w/w | **Ingredient** | **Chemical Name** |
|---|---|---|
| **Part A** | | |
| **6** | **Product of Example 4** | |
| 2 | PARSOL 1789 | Butyl Methoxydibenzoylmethane |
| 4 | PARSOL 5000 | 4-Methylbenzylidene camphor |
| 6 | Parsol MCX | Ethylhexyl methoxicinnamate |
| 2 | Uvinul T 150 | Ethylhexyltriazone |
| 1 | Silicone DC 200/350 cs | Dimethicone |
| 2 | Lanette O | Cetylstearyl alcohol |
| 3 | Softisan 100 | Hydrogenated coco-glycerides |
| 6 | Tegosoft TN | C12-15 Alkyl benzoate |
| 7 | Cetiol B | Dibutyl adipate |
| 2 | VITAMIN E ACETATE | Tocopheryl acetate |
| 1 | Berkemyol (Grape Seed) | Palmitoyl Grape seed Extract |
| 0.05 | BHT | Butylhydroxytoluol |
| 0.10 | Edeta BD | Disodium EDTA |
| 0.60 | Phenonip | Phenoxyethanol & Methylparaben & Ethylparaben & Propylparaben & Butylparaben |
| 2 | AMPHISOL | Cetyl phosphate DEA |

| **Part B** | | |
|---|---|---|
| ad 100 | Water deionized | Water deionized |
| 5 | Propylene Glycol | Propylene glycol |
| 0.30 | Carbopol 980 | Carbomer |

| **Part C** | | |
|---|---|---|
| 1.5 | KOH (10% sol.) | Potassium Hydroxide |

## Claims

1. Polysiloxanes **characterized by** the following structural elements per molecule:
one element of formula (H₃C)₃-Si- (I),
one element of formula -O-Si(CH₃)₃ (II),
2 to 200, elements in arbitrary order which are either identical or different from each other selected from the group consisting of formulae
-O-Si(CH₃)[CH(CH3)R¹]- (IIIa),
-O-Si(CH₃)(CH₂-CH₂-R¹)- (IIIb),
-O-Si(CH₃)[C(=CH₂)R¹]- (IIIc),
and
-O-Si(CH₃)(CH=CH-R¹)- (IIId);
2 to 200, elements in arbitrary order which are either identical or different from each other selected from the group consisting of formulae
-O-Si(CH₃)[CH(CH₃)R²]- (IVa),
-O-Si(CH₃)(CH₂-CH₂-R²)- (IVb),
-O-Si(CH₃)[C(=CH₂)R²]- (IVc),
and
-O-Si(CH₃)(CH=CH-R²)- (IVd);
optionally 1 to 100, elements in arbitrary order which are either identical or different from each other selected from the group consisting of formulae
-O-Si(CH₃)[CH(CH₃)R³]- (Va),
-O-Si(CH₃)(CH₂-CH₂-R³)- (Vb),
-O-Si(CH₃)[C(=CH₂)R³]- (Vc),
and
-O-Si(CH₃)(CH=CH-R3)- (Vd);
and optionally 1 - 20 elements in arbitrary order of formula -O-SiH(CH₃)- (VI)
wherein R¹ is a UV light absorbing group;
R² is hydrogen or a lipophilic group;
R³ is a group which is able to form ionogenic or hydrogen bonds.

2. Polysiloxanes according to claim 1 wherein the number of elements of formulae III is 5 to 80.

3. Polysiloxanes according to claim 1 or claim 2 wherein no elements of formulae V are present.

4. Polysiloxanes according to anyone of claims 1 - 3 wherein no elements of formula VI are present.

5. Polysiloxanes according to anyone of claims 1 - 4 wherein all substituents R¹ are identical.

6. Polysiloxanes according to anyone of claims 1 - 4 wherein at least two different types of substituents R¹ are present.

7. Polysiloxanes selected from the group consisting of the compounds represented by the following 9 formulae:

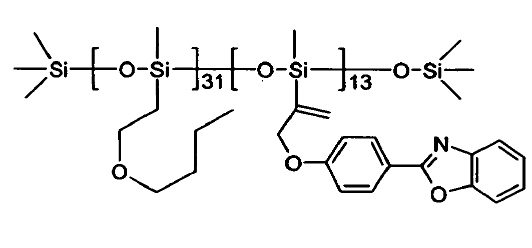

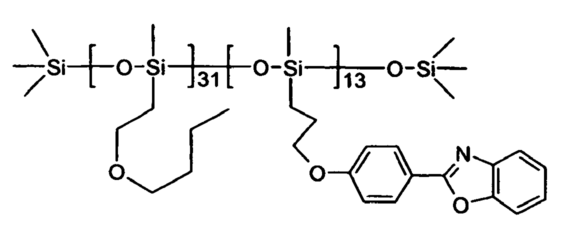

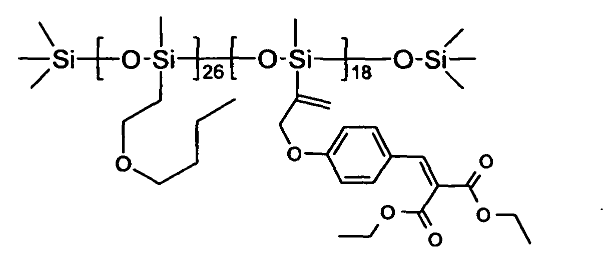

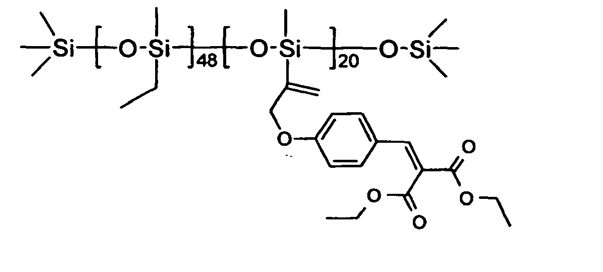

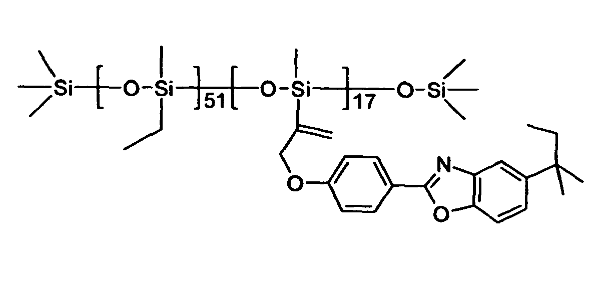

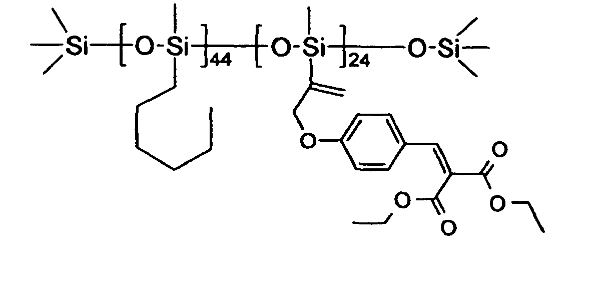

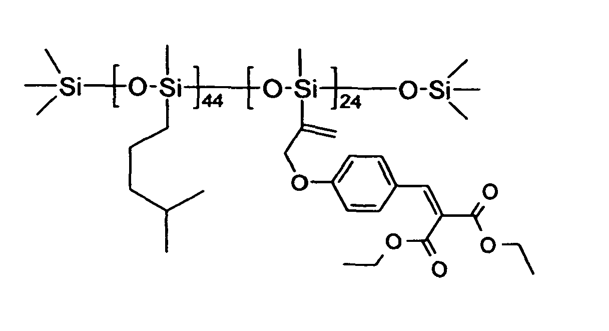

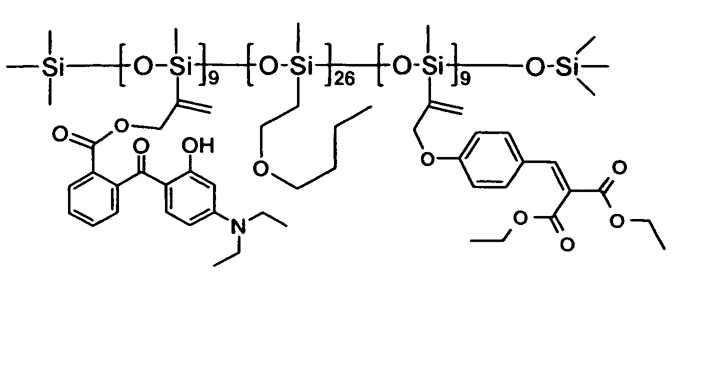

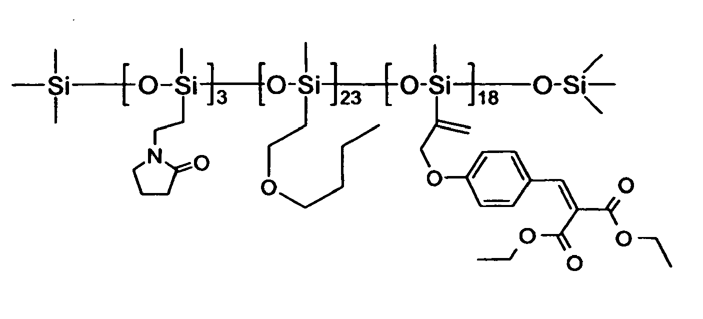

8. The use of a polysiloxane according to anyone of claims 1 - 7 as a sunscreen.

9. The use of a polysiloxane according to claim 8 for the protection of human skin or human hair.

10. Compositions comprising polysiloxanes according to anyone of claims 1 - 7 and at least one pharmaceutically and/or cosmetically acceptable excipient.

11. Compositions according to claim 10 comprising in addition at least one other UV light protective agent.

12. Compositions according to claims 10 and 11 for topical application.

## Patentansprüche

1. Polysiloxane, **gekennzeichnet durch** die folgenden Strukturelemente pro Molekül:
ein Element der Formel (H₃C)₃-Si- (I),
ein Element der Formel -O-si-(CH₃)₃ (II),
2 bis 200 Elemente in beliebiger Reihenfolge, die entweder gleich oder voneinander verschieden sind und aus der Gruppe bestehend aus den Formeln
-O-Si(CH₃)[CH(CH₃)R¹]- (IIIa),
-O-Si(CH₃)(CH₂-CH₂-R¹)- (IIIb),
-O-Si(CH₃)[C(=CH₂)R¹]- (IIIc)
und
-O-Si(CH₃)(CH=CH-R¹)- (IIId)
ausgewählt sind;
2 bis 200 Elemente in beliebiger Reihenfolge, die entweder gleich oder voneinander verschieden sind und aus der Gruppe bestehend aus den Formeln
-O-Si(CH₃)[CH(CH₃)R²]- (IVa),
-O-Si(CH₃)(CH₂-CH₂-R²)- (IVb),
-O-Si(CH₃)[C(=CH₂)R²]- (IVc)
und
-O-Si(CH₃)(CH=CH-R²)- (IVd)
ausgewählt sind; gegebenenfalls 1 bis 100 Elemente in beliebiger Reihenfolge, die entweder gleich oder voneinander verschieden sind und aus der Gruppe bestehend aus den Formeln
-O-Si(CH₃)[CH(CH₃)R³]- (Va),
-O-Si(CH₃)(CH₂-CH₂-R³)- (Vb),
-O-Si(CH₃)[C(=CH₂)R³]- (Vc)
und
-O-Si(CH₃)(CH=CH-R³)- (Vd)
ausgewählt sind;
und gegebenenfalls 1 - 20 Elemente in beliebiger Reihenfolge der Formel -O-SiH(CH₃)- (VI),
worin R¹ für eine UV-Licht absorbierende Gruppe steht;
R² für Wasserstoff oder eine lipophile Gruppe steht;
R³ für eine Gruppe steht, die zur Ausbildung von ionogenen Bindungen oder Wasserstoffbrückenbindungen befähigt ist.

2. Polysiloxane nach Anspruch 1, worin die Zahl der Elemente der Formeln III 5 bis 80 beträgt.

3. Polysiloxane nach Anspruch 1 oder Anspruch 2, worin keine Elemente der Formeln V vorliegen.

4. Polysiloxane nach einem der Ansprüche 1 - 3, worin keine Elemente der Formel VI vorliegen.

5. Polysiloxane nach einem der Ansprüche 1 - 4, worin alle Substituenten R¹ gleich sind.

6. Polysiloxane nach einem der Ansprüche 1 - 4, worin mindestens zwei verschiedene Typen von Substituenten R¹ vorliegen.

7. Polysiloxane aus der Gruppe bestehend aus den Verbindungen, die durch die folgenden 9 Formeln wiedergegeben werden:

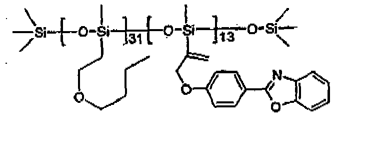

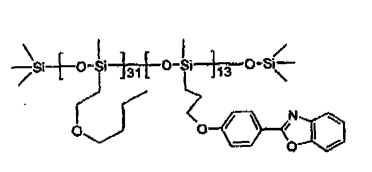

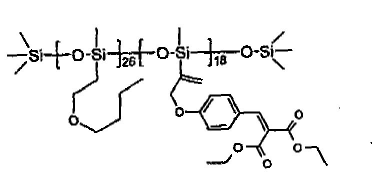

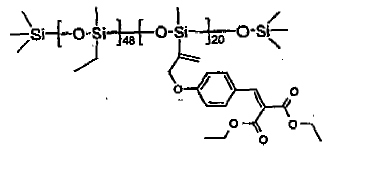

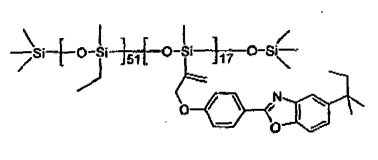

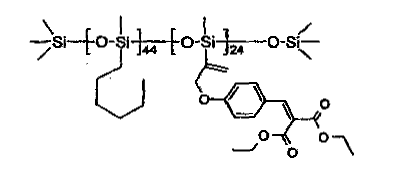

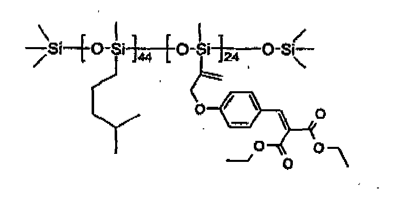

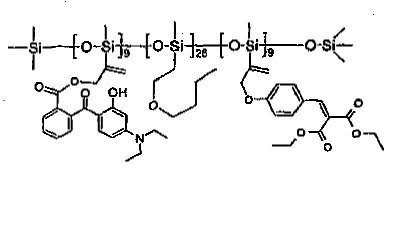

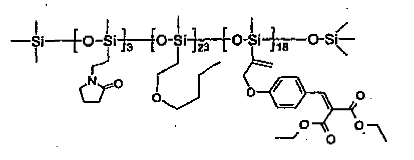

8. Verwendung eines Polysiloxans nach einem der Ansprüche 1 - 7 als Sonnenschutzmittel.

9. Verwendung eines Polysiloxans nach Anspruch 8 zum Schutz von menschlicher Haut oder menschlichem Haar.

10. Zusammensetzungen, enthaltend Polysiloxane nach einem der Ansprüche 1 - 7 und mindestens einen pharmazeutisch und/oder kosmetisch unbedenklichen Hilfsstoff.

11. Zusammensetzungen nach Anspruch 10, die außerdem mindestens ein anderes UV-Lichtschutzmittel enthalten.

12. Zusammensetzungen nach den Ansprüchen 10 und 11 für die topische Anwendung.

## Revendications

1. Polysiloxanes, **caractérisés par** les éléments de structure suivants par molécule :
un élément de formule (H₃C)₃-Si- (I),
un élément de formule -O-Si(CH₃)₃ (II),
de 2 à 200, éléments dans un ordre arbitraire qui sont soit identiques soit différents les uns des autres, choisis dans le groupe constitué des formules
-O-Si(CH₃)[CH(CH₃)R¹]- (IIIa),
-O-Si(CH₃)[CH₂-CH₂-R¹]- (IIIb),
-O-Si(CH₃)[C(=CH₂)R¹]- (IIIc),
et
-O-Si(CH₃)(CH=CH-R¹)- (IIId) ;
de 2 à 200, éléments dans un ordre arbitraire qui sont soit identiques soit différents les uns des autres, choisis dans le groupe constitué des formules
-O-Si(CH₃)[CH(CH₃)R²]- (IVa),
-O-Si(CH₃)(CH₂-CH₂-R²)- (IVb),
-O-Si(CH₃)[C(=CH₂)R²]- (IVc),
et
-O-Si(CH₃)(CH=CH-R²)- (IVd) ;
éventuellement de 1 à 100, éléments dans un ordre arbitraire qui sont soit identiques soit différents les uns des autres, choisis dans le groupe constitué des formules
-O-Si(CH₃)[CH(CH₃) R³]- (Va),
-O-Si(CH₃)(CH₂-CH₂-R³)- (Vb),
-O-Si(CH₃)[C(=CH₂)R³]- (Vc),
et
-O-Si(CH₃)(CH=CH-R3)- (Vd) ;
et éventuellement de 1 à 20 éléments dans un ordre arbitraire de formule -O-SiH(CH₃)- (VI)
dans lesquelles R¹ est un groupement absorbant la lumière UV ;
R² est hydrogène ou un groupement lipophile ;
R³ est un groupement qui est susceptible de former des liaisons ionogènes ou hydrogènes.

2. Polysiloxanes selon la revendication 1, **caractérisés en ce que** le nombre d'éléments de formules III est de 5 à 80.

3. Polysiloxanes selon la revendication 1 ou la revendication 2, **caractérisés en ce qu'**aucun éléments de formules V n'est présent.

4. Polysiloxanes selon l'une quelconque des revendications 1 à 3, **caractérisés en ce qu'**aucun éléments de formule VI n'est présent.

5. Polysiloxanes selon l'une quelconque des revendications 1 à 4, **caractérisés en ce que** tous les substituants R¹ sont identiques.

6. Polysiloxanes selon l'une quelconque des revendications 1 à 4, **caractérisés en ce qu'**au moins deux types différents de substituants R¹ sont présents.

7. Polysiloxanes choisis dans le groupe constitué des composés représentés par les 9 formules suivantes :

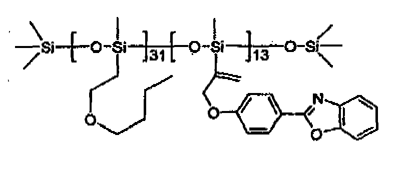

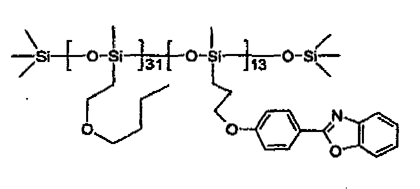

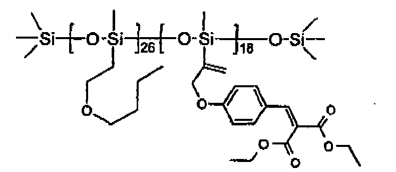

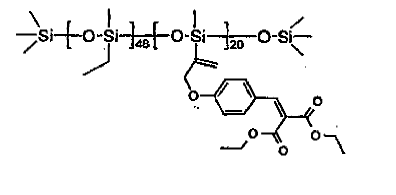

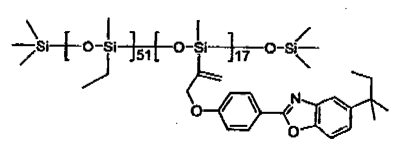

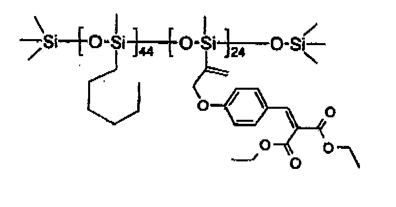

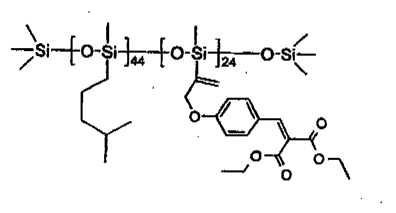

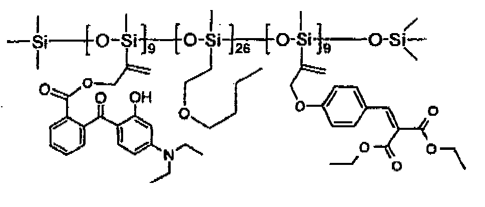

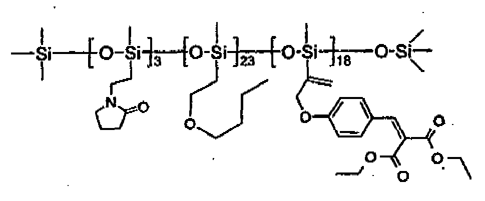

8. Utilisation d'un polysiloxane selon l'une quelconque des revendications 1 à 7, comme filtre solaire.

9. Utilisation d'un polysiloxane selon la revendication 8, pour la protection de la peau humaine ou des cheveux humains.

10. Compositions comprenant les polysiloxanes selon l'une quelconque des revendications 1 à 7 et au moins un excipient pharmaceutiquement et/ou cosmétiquement acceptable.

11. Compositions selon la revendication 10, comprenant en outre au moins un autre agent protecteur contre la lumière UV.

12. Compositions selon les revendications 10 et 11, destinées à l'application topique.
